# EUROPEAN PATENT APPLICATION

(11) **EP 3 299 083 A1**
(43) Date of publication of application: **28.03.2018**
(21) Application number: 16190468.5
(22) Date of filing: 23.09.2016
(51) Int. Cl.: B01J 13/02, A01N 25/28, A61K 8/11, A61K 9/50, B01J 13/18, C11D 3/50

(54) **CUSTOM-TAILORED SOL-GEL DERIVED MATRIXES FOR CHEMICAL IMMOBILIZATION**

(71) Applicant: Greenseal Research Ltd, 1600-542 Lisboa (PT)
(72) Inventor: LOPES MARQUES, Ana Clara, 1885-094 Moscavide-Portela (PT); DE JESUS VEGA LOUREIRO, Mónica, 2330-161 Entroncamento (PT); RIBEIRO SILVA GERALDES, Elisabete, 2695-602 São João Da Talha (PT); MOURA BORDADO, João Carlos, 1000-268 Lisboa (PT)
(74) Representative: BiiP cvba

(57) **Abstract**

A process for preparing microcapsules having a core-shell structure or interconnected porous microsphere matrix, and wherein said shell or matrix comprises at least one metal oxide and/or inorganic polymer, the microcapsules being abtained by a sol-gel process which comprises: preparing a first solution being a water-in-oil emulsion adding to said emulsion a second acidic aqueous pre-hydrolyzed metal precursor solution

## Description

### Field of the invention:

Encapsulation or entrapment, with or without covalent binding, of different compounds is an evolving area in chemistry with a significant importance in many industrial sectors, such as pharmaceutical, agrochemical, food, textile and cosmetic industries.

### Background of the invention:

Microparticles such as microcapsules, microspheres (MSs), micro scaffolds, or matrixes, in general, are used for storing and isolation of functional compounds, either gases, liquids or solids, which can, in a controlled fashion, release their valued payload.

Organic compounds have been widely used as encapsulating materials although polymer-based MSs normally suffer from poor chemical and physical stability and cannot be applied in certain environments. Therefore, research has been devoted to sol-gel derived inorganic metal oxide matrixes. The increased interest in inorganic matrixes is due to their distinct characteristics, such as the non-toxic quality for the environment, biocompatibility and the ability to easily incorporate additional functional groups, which enables the use of these structures in a variety of applications. Also, inorganic metal oxide matrixes present an increased chemical resistance plus a thermal and mechanical stability, when in comparison with polymeric matrixes.

Sol-gel technology combined with the micro emulsion method has been shown to be the most effective and economical technique for silica-based microspheres, microcapsules, micro scaffolds and other matrixes' synthesis. This method allows the synthesis of both inorganic and hybrid structures, while controlling the matrixes' microstructure, morphology, size, chemical composition, etc, and at the same time enabling low processing temperatures, proving, therefore, to be a versatile and cost-saving process. Microapsules composed of a shell prepared by a sol-gel process has been described in various publications: US patent Nos. 6,303,149, 6,238,650, 6,468,509, 6,436,375, US2005037087, US2002064541, and International publication Nos. WO 00/09652, WO00/72806, WO 01/80823, WO 03/03497, WO 03/039510, WO00/71084, WO05/009604, and WO04/81222, disclose sol-gel microcapsules and methods for their preparation. EP 0 934 773 and U.S. Pat. No. 6,337,089 teach microcapsules containing core material and a capsule wall made of organopolysiloxane, and their production. EP 0 941 761 and U.S. Pat. No. 6,251,313 also teach the preparation of microcapsules having shell walls of organopolysiloxane.

Known drawbacks of sol-gel doped matrices are long synthesis methods and the fact that said matrices cannot support high loading (of up to 95% wt.) of the active ingredient. In order to obtain high loading, it is essential to form a core-shell structure, where most of the weight of the capsule is the weight of the encapsulated active ingredient (core), and where the thin shell protects the core effectively

There is thus a widely recognized need for, and it would be highly advantageous to have active ingredients encapsulated/immobilized in sol-gel microcapsules, which are designed to stabilize and deliver the active ingredients encapsulated therein in an efficient and cost effective manner in many other applications where high loading of an encapsulated active ingredient in the composition is required.

Additionally it will be highly advantageous to have an efficient encapsulation method which is simplified in production and lower in cost and which is capable of having high concentrations of the core material (active ingredient) and yet preventing the leaching of the active ingredient from the microcapsules. Such a method will facilitate the encapsulation of a wide variety of molecules or substances, where the application may demand high loading of the encapsulated molecules or substances.

### Detailed description of the invention:

The present invention is directed to sol-gel microcapsules which encapsulate active ingredients, systems for topical application and compositions containing the active ingredients, methods of releasing, thereby delivering the active ingredients from the composition, methods of preparing the sol-gel microcapsules. Specifically, the microcapsules and/or compositions of the present invention are designed to stabilize the encapsulated active ingredients prior to application and/or to release the active ingredients after application and thus serve as a system for enhancing the stability of the active ingredient and/or as a delivery system.

In the present invention the term "core" refers to the inside part of the microcapsules comprising an active ingredient that is surrounded by the shell of the microcapsules.

In the present invention, the term "active ingredient" refers to any molecule or substance that can be used in agriculture, industry (including food industry), medicine, cosmetics, and which grants the final product (coating, cosmetics, pesticide, drug, etc.) at least one desired property.

According to a first embodiment, the present invention is directed to a process for preparing microcapsules having a core-shell structure or interconnected porous microsphere matrix, and wherein said shell or matrix comprises at least one metal oxide or inorganic polymer, the microcapsules being obtained by a sol-gel process which comprises: preparing a first solution being a water-in-oil emulsion adding to said emulsion a second acidic aqueous pre-hydrolyzed metal precursor solution

According to a further embodiment, the microcapsule of the present invention containing active ingredient is processed by a sol-gel process which comprises: preparing a first solution being a water-in-oil emulsion comprising said active ingredientadding to said emulsion a second acidic aqueous pre-hydrolyzed metal precursor solution

According to a alternative embodiment, microcapsules containing active ingredient in accordance with th epresent invention are synthesized by a sol-gel process which comprises: preparing a first solution being a water-in-oil emulsion adding to said emulsion a second acidic aqueous pre-hydrolyzed metal precursor solutionand whereby the active ingredient is entrapped/immobilized after the synthesis, via impregnation of the active ingredient assisted by a low vacuum process

Preferred metal precursor is a Si precursor, preferably selected from TEOS, TMOS, methylsilane (e.g. MTES, MTMS), glycidyloxysilane (e.g. GPTMS), methacryloxysilane (e.g. MPS), phenylsilane (e.g. PTES), aminosilane (e.g. APTMS, APTES), vinylsilane (e.g. VTES), sulphursilane and mixtures thereof

Highly preferred Si precursor is selected from TEOS, MTES, GPTMS and mixtures thereof resulting in matrices having different morphology, porosity and organic functionality characteristics which enable a customized immobilization or entrapment of a variety of active ingredients of different size. As a result, said matrices are suitable for different applications such as as bio carriers or as chemical storage.

Matrices with reduced levels of inner mesoporosity have been found especially in case whereby GPTMS is used as a precursor leading to particle structure enabling storage of macromolecules between aggregated particles acting as a scaffold for active ingredient immobilization.

Typically, the amount of precursor is more than 10% by total weight of the shell, preferably from 50 to 75% by total weight of the shell.

According to the present invention refers to the entrapment of different sized biocides, drugs and other active chemicals into previously synthetized microcapsules or porous microspheres inorganic silica or organic functional silica based microscaffolds, or matrixes. The microcapsules have organic functionalities, such as methyl, amino, phenyl, or epoxy functionality, different degrees of porosity (defined as micro, meso and macro porosity), morphologies and sizes. The referred microcapsules are synthesized using sol-gel processing technology in combination with the microemulsion technique, using Si precursors, including but not limited to Tetraethyl orthosilicate (TEOS), (3-Glycidyloxypropyl) trimethoxysilane (GPTMS) or/and methyltriethoxysilane (MTES), tetramethylorthosilicate (TMOS), methyltrimethoxysilane (MTMS), 3-(2-Aminoethylamino) propyltrimethoxysilane, 3-methacryloxypropyl trimethoxysilane (MPS), phenyltriethoxysilane (PTES), amino-propyl-trimethoxy silane (APTS), vinyltriethoxy silane (VTES) and mixtures thereof.

The entrapment of the active ingredient in the matrixes of the microcapsules occur due to physical entrapment in the different sized porosities or/and due to a chemical reaction of the active ingredient with the organic functional groups of the matrix. In the case of entrapment due to chemical reaction, the compounds might not only be entrapped in the different sized spaces of the matrixes' porosity but also have reacted with the surface. For example, the entrapment of an active ingredient chemical compound containing a primary, or secondary amine group in its composition, into a matrix obtained with an epoxy silane, i.e. containing an oxirane ring in its composition, might occur not only due to physical entrapment but also through reaction between the amine and the epoxy group of the matrix. In this case, it is possible to use the 'loaded' matrixes not only for an application where a slow release of the entrapped compound is needed, but also for an application in which the compound loss is unwanted, i.e. an anchored active chemical is desired. In this case a hybrid performance can be achieved, i.e by contact and by chemical release, simultaneously. This can lead for a longer-lasting and efficient performance (e.g. long-lasting and efficient anti-fouling performance)

Chemicals and biomolecules may be entrapped during the matrixes' synthesis process, by adding the active compound to be encapsulated either into the dispersed phase of the emulsion system, and/or in a subsequent step (i.e. after the matrix synthesis), with the aid of a low vacuum system, within the spaces of the porous, or worm-like structures (such as those achieved mainly for the TEOS-GPTMS and TEOS-MTES-GPMS derived matrixes, described in the examples).

### STRUCTURES

### • Low vacuum entrapment process:

Initially, the matrixes are subjected to a 400 mbar vacuum for 10 minutes, in order to extract the air present in the scaffolds' porosity. Afterwards, the matrixes are immersed within a solution of the biocide, drug or active chemical to be entrapped, and subjected to an ultrasonic bath for 30 minutes. Thereafter, the matrix with the load solution is subjected to a 200 mbar vacuum for 1 hour.

In the case of entrapment through chemical reaction of the active compound with reactive groups of the matrix, an additional step is preferred. The 'loaded' matrix should be subjected to a heat treatment (e.g. at 120°C for 1 hour, depending the reactions we want to promote), in order to secure a complete reaction between the active compound and the matrix. An additional filtration/washing step might be conducted, if necessary, with organic solvent of the entrapped compound. This additional filtration/washing step is devoted to removing the physically entrapped species at the surface of the matrixes, leaving only the chemically bonded (grafted) species.

### Matrixes preparation involving silane combinations

Referred Si precursors have been used in the different combinations presented in Table 1. High molar content of methyl silane and glycidyloxy silane (MTES and GPTMS) in combination with TEOS, for the preparation of such matrixes were used.

**Table 1 - Silanes' molar percentage**

| **Sample** | **Silanes (mol%)** | | |
|---|---|---|---|
| | **TEOS** | **MTES** | **GPTMS** |
| Combination 1 | 46 | 54 | 0 |
| Combination 2 | 53 | 0 | 47 |
| Combination 3 | 24 | 55 | 21 |

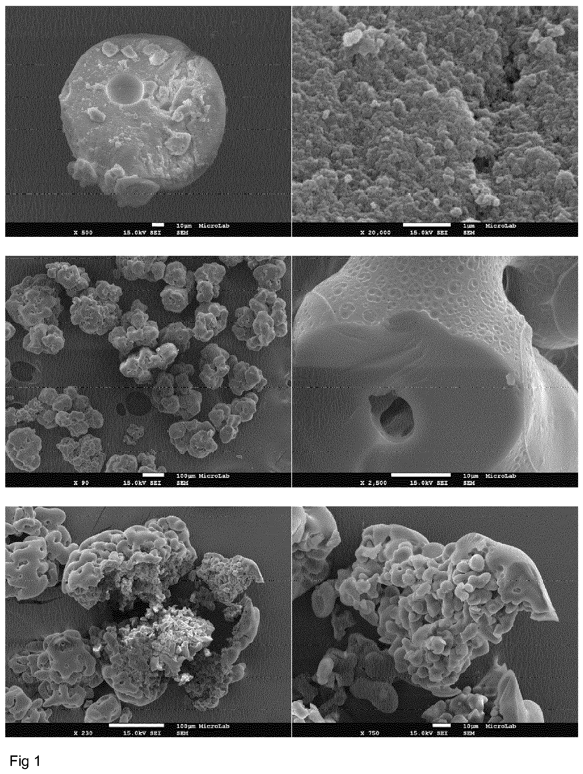

Figure 1 shows some matrixes obtained using the combinations 1, 2 and 3 referred in the Table 1, respectively and represents SEM images of matrixe samples obtained with the combination1, 2 and 3, respectively, with different magnifications
   TEOS-MTES-GPTMS scaffold (Combination 3)
   (Si precursors: **tetraethyl orthosilicate (TEOS), methyl triethoxysilane (MTES) and** 3-glycidoxypropyltrimethoxysilane (GPTMS)) *SURFACE:* (open porosity of ca. 10 µm size "pores" and mesopores)
Figure 2 shows SEM photomicrographs of the microscaffolds' surface, derived for the following Si precursors: **tetraethyl orthosilicate (TEOS), methyl triethoxysilane (MTES) and** 3-glycidoxypropyltrimethoxysilane (GPTMS), showing an open porosity, worm, or coral-like morphology. (Combination 3)
Figure 3 shows TEOS-MTES-GPTMS scaffold (Combination 3)
   *INTERIOR:* (ca. 1-5 µm size "pores"- open porosity - and mesopores). OH, methyl, epoxy organic functionality.
   SEM photomicrographs of the microscaffolds' interior, derived for the following Si precursors: tetraethyl orthosilicate (TEOS), methyl triethoxysilane (MTES) and 3-glycidoxypropyltrimethoxysilane (GPTMS), showing an open porosity, worm, or coral-like morphology, together with mesoporosity, typical of the materials obtained by the sol-gel method. (Combination 3)

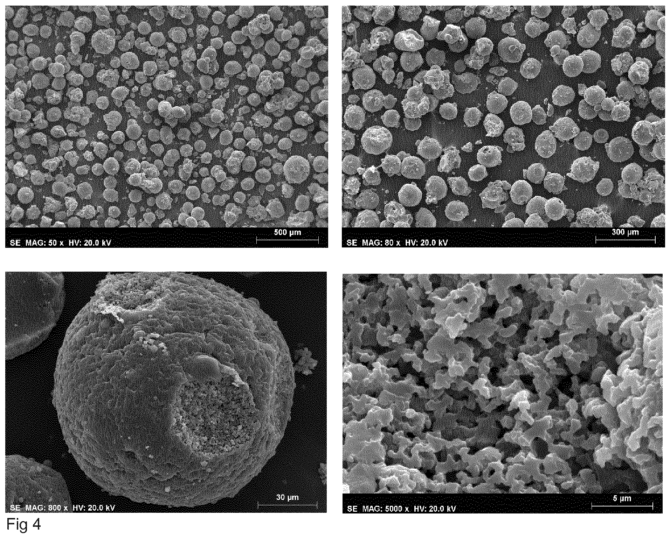

Figure 4 shows TEOS-GPTMS scaffold (Combination 2)
   OH, epoxy organic functionality.
   SEM photomicrographs of the spheric microscaffolds, derived for the following Si precursors: tetraethyl orthosilicate (TEOS) and 3-glycidoxypropyltrimethoxysilane (GPTMS), showing an open porosity, worm, or coral-like morphology, in the interior. (combination 2)

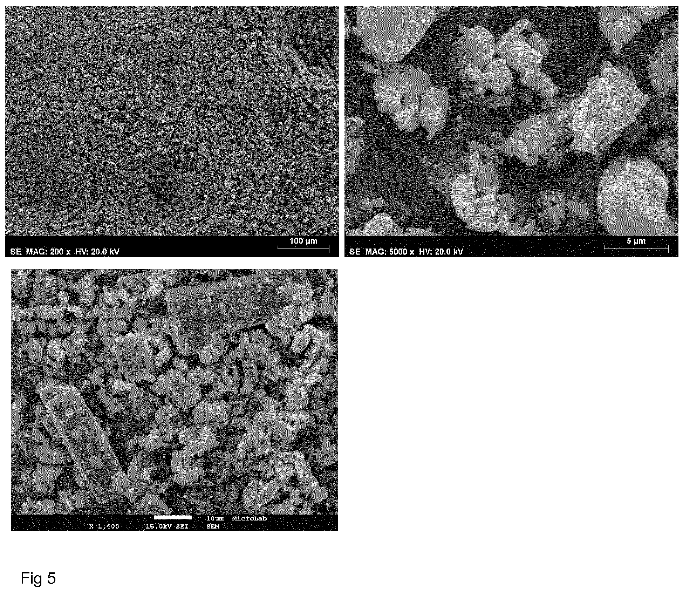

Figure 5 shows SEM photomicrographs of biocide Econea, showing its particle size, which fits well within the holes and surface porosity of the matrixes (microscaffolds)

### Example 1. - Entrapment of the biocide Econea® Technical Powder

The adhesion and growth of microorganisms on surfaces in contact with water is one of the most serious problems in water-immersed structures. There is a need for more environmental friendly anti-fouling strategies, able to combine a more efficient, long-lasting antifouling performance, together with non-toxic properties. Biocide Econea has been recently approved by EU Regulator (BPR), as a safe biocide and, in order to achieve a long-lasting antifouling effect, we have entrapped and partially grafted it on these microscaffolds, through reaction between its secondary amine and the oxirane ring of the silica-epoxy scaffold.

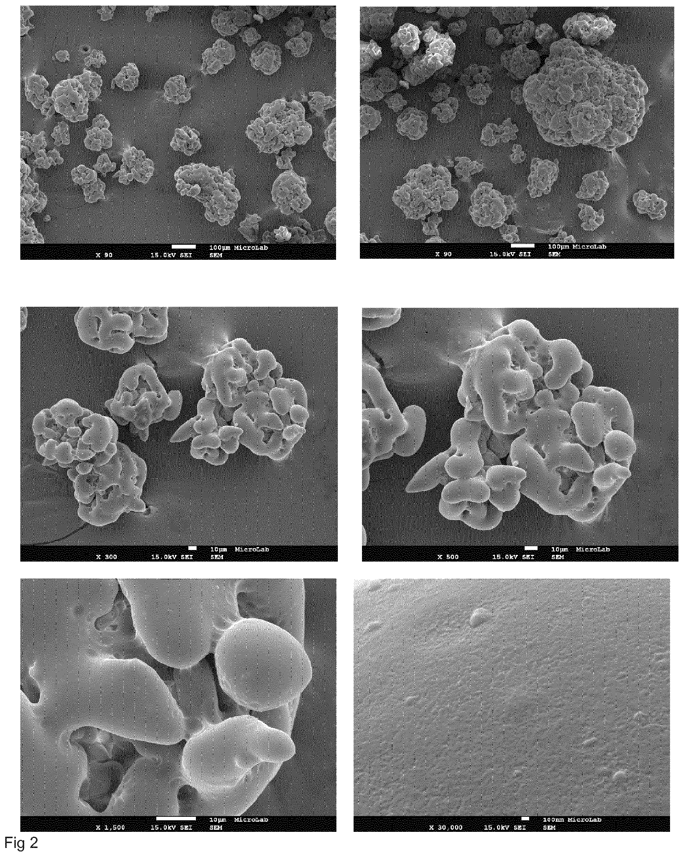

For the entrapment of the biocide Econea^{®} the matrixes with the combination 2 were used. The referred biocide has NH groups, as observed in Figure 2, which are reactive with the epoxy group of the GPTMS molecule present in this matrix.

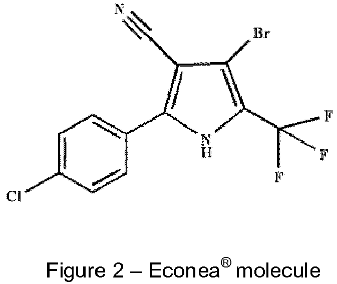

Initially the Econea^{®} powder was diluted in ethyl acetate, in a proportion of 2:3 m/m%.

The matrixes were subjected to a previous heat treatment at 100°C for 1 hour, in order to lose some possible remaining water that is still present from the synthesis process or gained, from ambient moisture, during handling.

The entrapment process was conducted as described above in the "Low vacuum entrapment process" section. The additional filtration/washing step was only conducted in half of the 'loaded' matrixes, for terms of comparison.

Both matrixes with Econea^{®}, the filtrated and not filtrated ones, were characterized through Fourier transform infrared spectroscopy, FTIR, using the Attenuated Total Reflectance accessory, Thermogravimetric analysis, TGA and Scanning electron microscopy with energy dispersive x-ray (SEM-EDS).

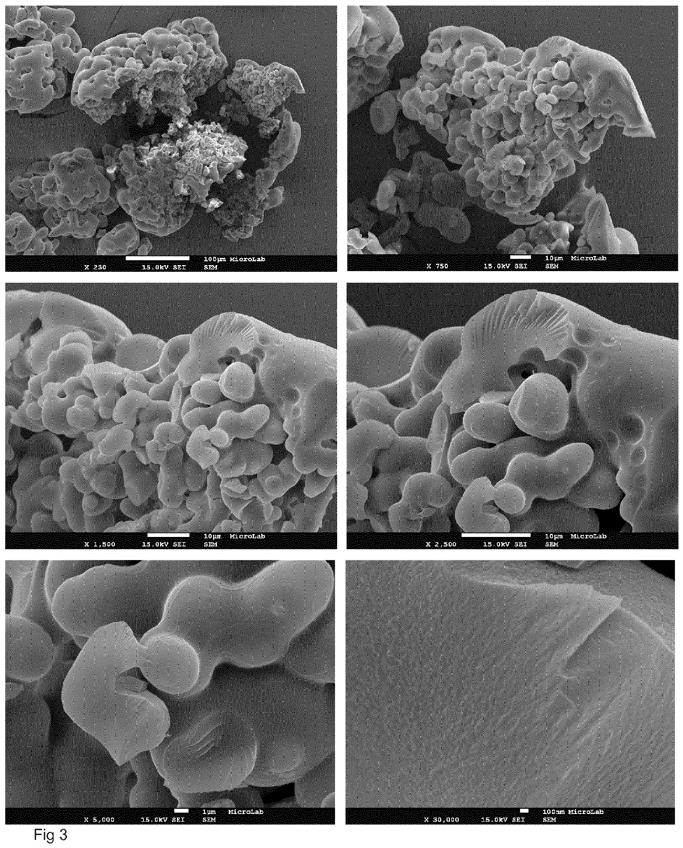

The presence of Econea within the microscaffolds is revealed by FTIR (Figure 3), thermogravimetric analyses (TGA) (Figure 4) and scanning electron microscopy (SEM-EDX). The release of Econea from the scaffolds when immersed into salted water was followed by TGA and their biocidal effect was assessed against Staphylococcus aureus bacteria at different mediums showing very promising results and revealing a hybrid antifouling performance both by contact and by release.

### Example 2. - Entrapment of peppermint essential oil

There is a need for a long-lasting perfume effect, or other substances long-lasting effect, or even for the entrapment and immobilization of harmful or toxic chemicals.

The silica-based microscaffolds herein disclosed entrap and promote a strong delay of essential oils, or perfumes.

Their refractive index is similar to that of the perfumes, and therefore they are invisible within the liquid perfume.

Moreover, they can be used as a charge, such as fumed silica in rubber formulations, or in other elastomeric, thermoplastic or thermoset formulations. Their retardant effect of essential oil release can be a plus e.g. in parts for shoes, vehicles, etc., giving a perfume sense to rubber products, or other plastics, and acting at the same time as a reinforcement charge, increasing the mechanical resistance, and fire resistance of plastic products.

For the entrapment of the essential oil, the matrix with the combination 2 was used. In this case, the compound to be entrapped is not reactive with the matrix, therefore the 'load' will only occur due to physical entrapment of the molecules within the spaces of the worm-like structures. The essential oils are composed of lipophilic and highly volatile secondary plant metabolites. Its entrapment in the matrix is found to retard its evaporation, leading to a more lasting effect.

The matrixes were subjected to a previous heat treatment at 100°C for 1h, in order to lose some possible remaining water that is still present from the synthesis process or gained, from ambient moisture, during handling.

The entrapment process was conducted as described above in the "Low vacuum entrapment process" section. The additional filtration/washing step was not conducted in this case.

The matrixes with essential oil were also characterized through FTIR and TGA.

The FTIR results demonstrate that it was possible to identify the essential oil in the 'loaded' matrixes, through the identification of its characteristic groups, not presented in the matrixes alone, which confirmed the success of its entrapment in the matrixes' porosity.

Isothermic TGA analysis was conducted and confirmed the essential oil having a lower tendency to evaporate, at a given temperature, when loaded into the scaffolds.

The essential oil without being loaded into the scaffolds presents a more accentuated decrease in its mass over time, indicating that the scaffolds are significantly retarding the essential oil evaporation and that the loss rate is clearly faster in the case of the essential oil alone, confirming the capability of the matrix to retard the oils' evaporation.

## Claims

1. A process for preparing microcapsules having a core-shell structure or interconnected porous microsphere matrix, and wherein said shell or matrix comprises at least one metal oxide and/or inorganic polymer, the microcapsules being obtained by a sol-gel process which comprises: preparing a first solution being a water-in-oil emulsion adding to said emulsion a second acidic aqueous pre-hydrolyzed metal precursor solution

2. A process for preparing microcapsules having a core-shell structure or interconnected porous microsphere matrix, wherein said core or pores include an active ingredient, and wherein said shell or matrix comprise at least one metal oxide and/or inorganic polymer, the microcapsules being obtained by a sol-gel process which comprises: preparing a first solution being a water-in-oil emulsion comprising said active ingredientadding to said emulsion a second acidic aqueous pre-hydrolyzed metal precursor solution

3. A process for preparing microcapsules having a core-shell structure or interconnected porous microsphere matrix, wherein said core or pores include an active ingredient, and wherein said shell or matrix comprise at least one metal oxide and/or inorganic polymer, the microcapsules being synthesized by a sol-gel process which comprises: preparing a first solution being a water-in-oil emulsion adding to said emulsion a second acidic aqueous pre-hydrolyzed metal precursor solution and whereby the active ingredient is entrapped/immobilized after the synthesis, via impregnation of the active ingredient assisted by a low vacuum process

4. A process according to claims 1-3 wherein said metal precursor is preferably a Si precursor, selected from TEOS, TMOS, methylsilane (e.g. MTES, MTMS), glycidyloxysilane (e.g. GPTMS), methacryloxysilane (e.g. MPS), phenylsilane (e.g. PTES), aminosilane (e.g. APTMS, APTES), vinylsilane (e.g. VTES), sulphursilane and mixtures thereof

5. A process according to claim 4 wherein said Si precursor is selected from TEOS, MTES, GPTMS and mixtures thereof.

6. A process according to claim 4 wherein said Si precursor is selected from GPTMS and mixtures thereof.

7. A process according to claims 1-6 whereby the amount of precursor is more than 10% by total weight of the shell, preferably from 50 to 75% by total weight of the shell.

8. A process according to claims 1-7 whereby said metal oxide is silicone oxide and/or silica

9. Active ingredient containing microcapsules having a core-shell structure or interconnected porous microsphere matrix, and wherein said shell or matrix comprises at least one inorganic polymer having organic functionality selected from methyl,amino,phenyl and epoxy or mixtures therof

10. Fragrance active ingredient containing microcapsules having a core-shell structure or interconnected porous microsphere matrix, and wherein said shell or matrix comprises at least one inorganic polymer having organic functionality selected from methyl,amino,phenyl and epoxy or mixtures therof

11. Biocide active ingredient containing microcapsules having a core-shell structure or interconnected porous microsphere matrix, and wherein said shell or matrix comprises at least one inorganic polymer having organic functionality selected from methyl,amino,phenyl and epoxy or mixtures therof

12. Biocide NH group active ingredient containing microcapsules having a core-shell structure or interconnected porous microsphere matrix, and wherein said shell or matrix comprises at least one inorganic polymer having epoxy functionality.

13. The use of active ingredient containing microcapsules as defined in claims 11 and 12 to provide anti fouling properties by contact and/or by release.

14. A polymeric composition comprising the microcapsules as defined in claim 11 and 12 including but not limited to a coating composition or a paint composition.

15. The use of active ingredient containing microcapsules having a core-shell structure or interconnected porous microsphere matrix, and wherein said shell or matrix comprises at least one inorganic polymer having organic functionality selected from methyl,amino,phenyl and epoxy or mixtures thereof ty to provide controlled release of said active ingredient
